(19) Europäisches Patentamt
European Patent Office
Office européen des brevets

(11) Numéro de publication : **0 494 010 A1**

(12) # DEMANDE DE BREVET EUROPEEN

(21) Numéro de dépôt : **91403498.8**

(22) Date de dépôt : **20.12.91**

(51) Int. Cl.[5] : **C07D 401/04,** C07D 409/14, A61K 31/38, A61K 31/415, A61K 31/445

(30) Priorité : **31.12.90 FR 9016540**

(43) Date de publication de la demande : **08.07.92 Bulletin 92/28**

(84) Etats contractants désignés : **AT BE CH DE DK ES FR GB GR IT LI LU NL SE**

(71) Demandeur : **INSTITUT NATIONAL DE LA SANTE ET DE LA RECHERCHE MEDICALE (INSERM)**
**101, rue de Tolbiac**
**F-75654 Paris Cédex 13 (FR)**
(71) Demandeur : **SOCIETE CIVILE BIOPROJET**
**30, rue des Francs Bourgeois**
**F-75003 Paris (FR)**
(71) Demandeur : **UNIVERSITE DE CAEN**
**Esplanade de la Paix**
**F-14032 Caen (FR)**

(72) Inventeur : **Arrang, Jean-Michel**
**Bât.11, Res.du Château de Courcelles, 160 Avenue**
**du Général Leclerc, F-91190 Gif/Yvette (FR)**
Inventeur : **Garbarg, Monique**
**10 rue Théodore de Banville**
**F-75017 Paris (FR)**
Inventeur : **Lancelot, Jean-Charles Maurice**
**Le Bourg**
**F-14400 Tour en Bessin (FR)**
Inventeur : **Lecomte, Jeanne-Marie**
**30 rue des Francs Bourgeois**
**F-75003 Paris (FR)**
Inventeur : **Robba, Max-Fernand**
**4 rue Massillon**
**F-75004 Paris (FR)**
Inventeur : **Schwartz, Jean-Charles**
**9 Villa Seurat**
**F-75014 Paris (FR)**

(74) Mandataire : **Bernasconi, Jean et al**
**CABINET LEMOINE ET BERNASCONI 13, Boulevard des Batignolles**
**F-75008 Paris (FR)**

(54) **Nouvelles 4-(4-imidazolyl) pipéridines substituées en 1, leur préparation ainsi que leurs applications thérapeutiques.**

(57) Les composés répondent à la formule générale

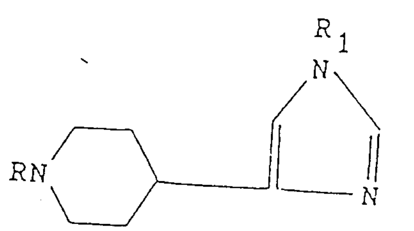

I

dans laquelle R $_1$ représente un atome d'hydrogène ou le groupement -COR $_2$ dans lequel R.$_2$ représente le noyau benzénique, les groupements cyclopentylméthyle, cyclohexylméthyle, cyclopentyléthyle ou cyclohexyléthyle, les groupements cyclopentylamine, cyclohexylamine, phénylamine, chlorophénylamine ou dichlorophénylamine ; R représente un atome d'hydrogène ou le groupement $COR_3$ dans lequel $R_3$ représente un groupement aliphatique, un noyau cyclanique ou benzénique, un groupement $(CH_2)_n$ $R_4$ , un groupement -CH = $CHR_8$, le groupe amine secondaire - $NH(CH_2)_nR_9$ ; R représente encore le groupement hydroxy alkényle :

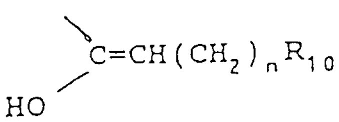

EP 0 494 010 A1

La présente invention concerne de nouvelles 4-(4-imidazolyl) pipéridines substituées en 1, leur préparation ainsi que leurs applications thérapeutiques.

Les composés conformes à l'invention répondent à la formule générale

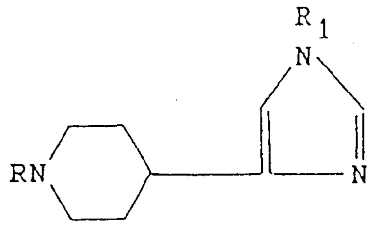

I

dans laquelle

$R_1$ représente un atome d'hydrogène ou le groupement $-COR_2$ dans lequel $R_2$ représente le noyau benzénique, les groupements cyclopentylméthyle, cyclohexylméthyle, cyclopentyléthyle ou cyclohexyléthyle, les groupements cyclopentylamine, cyclohexylamine, phénylamine, chlorophénylamine ou dichlorophénylamine ;

R représente un atome d'hydrogène ou le groupement $COR_3$ dans lequel $R_3$ représente

(a) un groupement aliphatique linéaire ou ramifié comportant 1 à 11 et notamment 1 à 9 atomes de carbone,

(b)un noyau cyclanique tel que cyclopropane, phénylcyclopropane, cyclobutane, cyclopentane,cyclohexane, cycloheptane, norbornane, adamantane, noradamantane, chlorooxonorbornane, chloroéthylène dioxynorbornane, bromoéthylène dioxynorbornane et le groupement anhydre de l'acide hydroxy carboxy 1,2,2-triméthylcyclopentane carboxylique,

(c) un noyau benzénique non substitué ou substitué en para par un groupement aliphatique linéaire ou ramifié comportant 3 à 5 atomes de carbone ainsi que par un halogène,

(d) un groupement $(CH_2)_nR_4$ dans lequel n est un nombre compris entre 1 et 10, et $R_4$ représente un noyau cyclanique tel que cyclopropane, cyclobutane, cyclopentane, cyclopentène, cyclohexane, cycloheptane, norbornane, noradamantane, adamantane et 6,6-diméthylbicyclo [3,1,1] heptène ; un cycle benzénique non substitué ou monosubstitué par un atome de fluor, un atome de chlore, un groupement méthyle ou un groupement méthoxy; le cycle thiophénique greffé par son sommet 2 ou son sommet 3 ; un groupement ester d'acide carboxylique $COOR_5$ dans lequel $R_5$ est un noyau cyclanique tel que cyclopropane, cyclobutane, cyclopentane, cyclohexane ou norbornane ; un groupement amide d'acide carboxylique de structure $CONHR_6$ dans lequel $R_6$ représente un noyau cyclanique tel que cyclopropane, cyclobutane, cyclopentane, cyclohexane ou norbornane ; un groupement amide d'acide carboxylique de structure 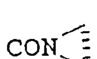 dans lequel le groupement 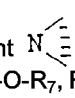 représente la pyrrolidine, la pipéridine, la 2,6-diméthylmorpholine ; un groupement éther oxyde $-O-R_7$, $R_7$ pouvant être un noyau benzénique non substitué ou monosubstitué par un atome de chlore ou de fluor ou disubstitué par un atome de chlore et par un groupement méthyle ;

(e) un groupement $-CH=CHR_6$ dans lequel $R_8$ représente un noyau cyclanique tel que cyclopropane, cyclobutane, cyclopentane, cyclohexane, norbornane ou norbornène ;

(f) le groupe amine secondaire $-NH(CH_2)_nR_9$ dans lequel n est un nombre compris entre 1 et 5 et $R_9$ constitue un noyau cyclanique tel que cyclopropane, cyclobutane, cyclopentane, cyclohexane ou norbornane, un noyau benzénique non substitué, monosubstitué par un atome de fluor ou de chlore ou par un groupement méthoxy ou trisubstitué par les groupements méthoxy ;

R représente encore le groupement hydroxy alkényle

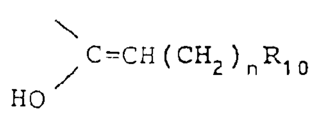

dans lequel n est un nombre compris entre 2 et 9 et $R_{10}$ représente le noyau benzénique ou le groupement phénoxy ; ainsi que le groupement

$$C\ S\ NH\ (CH_2)_n\ R_9$$

dans lequel n est un nombre compris entre 1 et 5 et $R_9$ a la signification précitée.

Les composés de formule I conformes à l'invention dans laquelle $R_1$ désigne l'hydrogène et R a la signification précitée, sont préparés par réaction de la 4-(4-imidazolyl)pipéridine de formule

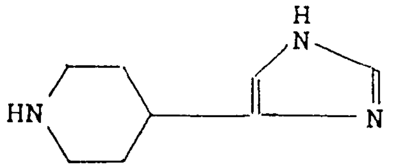

II

a) avec un chlorure d'acide $R_3$ COCl ou un anhydride mixte tel que $R_3$ $CO_2CO_2C_2H_5$ lorsqu'il s'agit d'obtenir des amides de formule générale

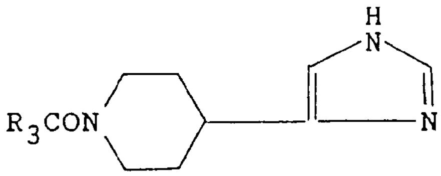

III

ou

b) lorsqu'il s'agit de préparer des urées de formule IV ou des thiourées de formule V

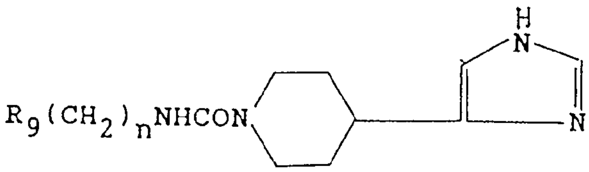

IV

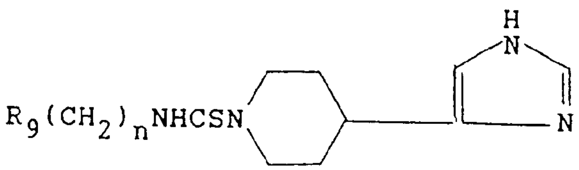

V

où n et $R_9$ ont la signification précitée, avec un isocyanate $R_9$ $(CH_2)_nNCO$, obtenu à partir d'un acide $R_9$ $(CH_2)_nCOOH$ par une modification de la synthèse de Curtius, ou avec un isothiocyanate $R_9$ $(CH_2)_nNCS$.

Les composés de formule I conformes à l'invention, dans laquelle $R_1$ désigne le groupement $COR_2$ et R a la signification précitée sont obtenus par réaction d'un composé de formule

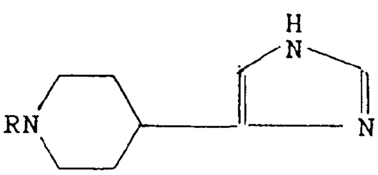

VI

où R a la signification précitée.

a) avec un chlorure d'acide $R_2COCl$ lorsqu'il s'agit d'obtenir un composé de formule

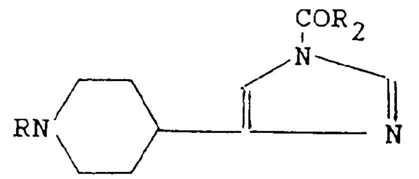

VII

où $R_2$ représente le noyau benzénique ou les groupements cyclopentylméthyle, cyclohexylméthyle, cyclopentyléthyle ou cyclohexyléthyle ;

ou

b) avec l'isocyanate correspondant lorsqu'il s'agit d'obtenir un composé de formule VII où $R_2$ représente les groupements cyclopentylamine, cyclohexylamine, phénylamine, chlorophénylamine ou dichlorophénylamine.

La réaction énoncée sous a) s'effectue par exemple par chauffage à une température de l'ordre de 80°C dans l'acétonitrile en présence de triéthylamine.

La réaction de condensation avec l'isocyanate ou isothiocyanate énoncée sous b) s'effectue de façon connue en soi, au reflux dans un solvant apolaire tel que le benzène.

Les composés conformes à l'invention répondant à la formule générale I peuvent être salifiés par les acides minéraux ou les acides organiques courants en vue de leur utilisation thérapeutique sous forme de sels.

Les exemples qui sont donnés, ci-après, à titre non limitatif, illustrent la présente invention.

Les exemples 1 à 7 illustrent la préparation des composés de formule générale I dans laquelle R a la signification précitée et $R_1$ représente l'hydrogène.

Les exemples 8 et 9 décrivent les méthodes générales de synthèse des isocyanates et des N-(4-imidazolyl)pipéridinylurées (composés de formule IV).

L'exemple 10 illustre la préparation d'une thiourée de formule V.

Les exemples 11 et 12 illustrent la préparation des composés de formule générale I dans laquelle R a la signification précitée et $R_1$ représente le groupement $COR_2$, $R_2$ ayant la signification précitée.

Exemple 1 :

1-(NORBORNYLMETHYLCARBONYL)-4-(1H-IMIDAZOLYL-4) PIPERIDINE (composé 23)

On ajoute goutte à goutte à 0°C à une solution de 2g (0,0132 mole) d'acide 2-norbornane-acétique dans 60 ml d'acétonitrile 2,03g (0,0132 mole) de triéthylamine. Après 30 minutes d'agitation, on ajoute goutte à goutte 1,43g (0,0132 mole) de chloroformiate d'éthyle de telle façon que la température reste comprise entre 0°C et 5°C. Après 30 minutes d'agitation, la solution est versée dans 60 ml d'acétonitrile et 15 ml d'eau contenant 1,99g (0,0132 mole) de 4-(imidazolyl-4)pipéridine. Le mélange réactionnel est chauffé 1 heure à 80°C. Après refroidissement, la solution est concentrée sous pression réduite. Le résidu huileux est repris dans 40 ml d'eau puis extrait par un mélange éther éthylique/acétate d'éthyle (60:40). La phase organique est séchée sur sulfate de magnésium, filtrée puis concentrée sous pression réduite. L'huile résiduelle cristallise par addition d'un mélange éther éthylique/éther de pétrole/hexane (20:5:5). On obtient, après recristallisation dans un mélange éther/éther de pétrole (60:40), une poudre blanche.

F = 118 - 120°C

Masse = 2,2g

Rendement = 58%

Spectre IR (KBr) ; bandes principales :

3120 (NH), 1635 (C = 0), 1450, 1310, 1275, 1205, 1030, 990 870, 770 et 645 $cm^{-1}$.

Analyse : $C_{17}H_{25}N_3O$

| | | | | | | | | | | |
|---|---|---|---|---|---|---|---|---|---|---|
| Calc % | : | C | : | 71,04 | H | : | 8,77 | N | : | 14,62 |
| Trouvé % | : | C | : | 71,19 | H | : | 8,63 | N | : | 14,79 |

Spectre de RMN$^1$H(DMSO-$d_6$) : H2 imidazole : 7,50 ppm ; H5 imidazole : 6,71 ppm ; H pipéridine et norbornyle 4,33, 3,90, 3,43, 2,76, 2,00, 1,26, ppm (sous forme de massifs).

Les composés 1 à 22, 24 à 58 et 62 à 66 (Tableau I) sont obtenus de façon analogue au composé 23.

Exemple 2 :

1-(NORBORNYL PROPIONYL CARBONYLE 4-(4-1H-IMIDAZOLYL) PIPERIDINE (Composé 24)

A. ACIDE 3-(2-NORBORNYL)-PROPIONIQUE

Une solution de 20g (0,129 mole) d'acide norbornyl-2-acétique et de 3 équivalents d'hydrure de lithium aluminium dans 300 ml d'éther anhydre est portée au reflux pendant 4 heures. Après hydrolyse de la solution, la phase éthérée est décantée, lavée à l'eau, séchée puis concentrée sous pression réduite. On obtient 14g (77%) de 2-(2-hydroxyméthyl)norbornane. Ces 14g (0,1 mole) d'alcool sont mis en solution dans 60 ml de pyridine à 0°C et on ajoute par petites fractions 38,10g (2 équivalents) de chlorure de tosyle. On agite 8H à température ordinaire. La solution est hydrolysée par 250 ml d'éther éthylique. La phase éthérée est décantée, séchée et concentrée sous pression réduite. L'huile résiduelle (masse = 23g, Rendement = 78%) est mise en solution dans 50 ml de DMSO en présence de 10g (0,153 mole) de cyanure de potassium et portée à 80°C pendant 2H. Après extraction de la solution par 500 ml d'éther éthylique, on obtient 7g (60%) de 3-(2-norbornyl)propionitrile.
Les 7g (0,046 mole) de nitrile sont chauffés au reflux dans 70 ml d'une solution aqueuse d'hydroxyde de sodium à 20%. Après refroidissement, la solution est extraite par 70 ml d'éther. Après décantation, la phase aqueuse est acidifiée par une solution d'acide chlorydrique concentrée. Après extraction avec 170 ml d'éther, on obtient 5g (64%) d'acide 3-(2-norbornyl)propionique sous forme d'une huile jaune pâle.
Pt. Eb. : 150°C / 5mm
Spectre IR (KBr) : (CO) 1700, bandes principales à 1410, 1280, 1220 et 930 $cm^{-1}$

| | | |
|---|---|---|
| Analyse : $C_{10}H_{15}O_2$ | | |
| | Calculé % : | C : 71,39 ; H : 9,58 |
| | Trouvé % : | C : 71,42 ; H : 9,60 |

B. 1-NORBORNYL PROPIONYL CARBONYL)-4-(4-1H-IMIDAZOLYL PIPERIDINE

On ajoute goutte à goutte à 0°C à une solution de 2,21g (0,0132 mole) du composé obtenu sous A dans 70 ml d'acétonitrile 2,03g (0,0132 mole) de triéthylamine. Après 30 minutes d'agitation, on ajoute 1,43g (0,0132 mole) de chloroformiate d'éthyle de telle façon que la température reste comprise entre 0°C et 5°C. Après 30 minutes d'agitation, la solution est versée dans 60 ml d'acétonitrile et 15 ml d'eau contenant 1,99g (0,0132 mole) de 4-(imidazolyl-4) pipéridine. Après 1 heure de chauffage à 80°C, la solution est concentrée sous pression réduite, le résidu huileux est repris par 20 ml d'eau puis extrait par 70 ml d'acétate d'éthyle. L'huile résiduelle obtenue cristallise par addition d'un mélange éther éthylique/éther de pétrole (30:20). On obtient après recristallisation dans un mélange acétate d'éthyle/éther (30:20) le composé du titre sous forme de poudre blanche.
F = 158°C
Masse = 2,6g
Rendement = 65%
Spectre IR (KBr) : (NH) 3130, (CO) 1640 $cm^{-1}$
Analyse : C, H, N.

Exemple 3 :

1[1'-(1'- HYDROXY- 4'- PHENYL BUTEN-1'-YL)]-4- (4'-IMIDAZOLYL)PIPERIDINE (BIS CHLORHYDRATE) (Composé 59)

On ajoute goutte à goutte à 0°C à une solution de 2,16g (0,0132 mole) d'acide 4-phénylbutyrique dans 70 ml d'acétonitrile 2,03g (0,0132 mole) de triéthylamine. Après 30 minutes d'agitation, on ajoute 1,43g (0,0132 mole) de chloroformiate d'éthyle de telle façon que la température reste comprise entre 0°C et 5°C. Après 30 minutes d'agitation, la solution est versée dans 60 ml d'acétonitrile et 15 ml d'eau contenant 1,99g (0,0132 mole) de 4-(imidazolyl-4)pipéridine. Après 1 heure de chauffage à 80°C, la solution est concentrée sous pression réduite, le résidu huileux est repris par 20 ml d'eau puis extrait par 70 ml d'acétate d'éthyle. L'huile résiduelle obtenue cristallise par addition d'un mélange éther éthylique/éther de pétrole (30:20). On obtient après recristallisation dans un mélange éther/hexane (30:20) une poudre blanche (composé 42).
F = 102°C
Masse = 3,2g
Rendement = 81 %

On ajoute ensuite 3,2g (0,81 mole) de la base obtenue (composé 42) dans 20 ml d'isopropanol en présence de 2,1 équivalents d'acide chlorydrique concentré. Le précipité est essoré, lavé avec 10 ml d'alcool isopropylique puis avec 15 ml d'éther éthylique. Après séchage, le précipité est recristallisé dans l'acétonitrile. On obtient le composé du titre sous forme de poudre blanche.
F = 126°C
Rendement = 55 %
Analyse : C, H, N, Cl. Recristallisation dans acétonitrile (70) + éther éthylique (30).

Exemple 4 :

1[1'-(1'- HYDROXY-11'- PHENOXY UNDECEN-1'-YL)]-4-(4'-IMIDAZOLYL)PIPERIDINE (BIS CHLORHYDRATE) (Composé 60)

Ce composé est obtenu selon le même procédé de synthèse que le composé 59 de l'exemple 3 à partir de la base 58 (Tableau 1).
F = 115°C
Rendement = 55 %
Analyse : C, H, N, Cl. Recristallisation dans acétonitrile (7) + éther éthylique (3).

Exemple 5 :

1-(3-PHENYLPROPIONYL)-4-(4- 1H- IMIDAZOLYL)- PIPERIDINE (OXALATE) (Composé 38)

0,5g (0,0017 mole) de 1-(3-phénylpropionyl)-4-(4-1H-imidazolyl)-pipéridine et 1,2 équivalent d'acide oxalique en solution dans 35 ml d'isopropanol sont portés au reflux 1H. Après refroidissement, le précipité obtenu est essoré, lavé à l'éther éthylique, séché et recristallisé dans l'acétonitrile. On obtient une poudre blanche.
F = 129°C
Masse = 0,59g
Rendement = 94 %
Analyse (C, H, N).

Exemple 6 :

1-(CYCLOHEXYLAMINO CARBONYL N-PENTANOYL-4-(1H-IMIDAZOLYL-4)PIPERIDINE (Composé 54)

A. MONO N-CYCLOHEXYLAMIDE DE L'ACIDE ADIPIQUE

On ajoute 34,8g (0,2 mole) d'ester monoéthylique de l'acide adipique goutte à goutte à 19,8g (0,2 mole) de cyclohexylamine sous agitation. La température monte à 60°C. Le melange réactionnel est chauffé à 80°C 1H. Après refroidissement, la solution est versée dans 200 ml d'eau puis extraite par 300 ml d'éther éthylique. La phase éthérée est agitée dans 150 ml d'une solution d'hydrogènocarbonate de potassium. Après décantation de la phase éthérée, la phase aqueuse est acidifiée jusqu'à pH 1 par l'acide chlorhydrique concentré. Le précipité est essoré, lavé à l'eau, séché et recristallisé dans l'acétonitrile.
Masse = 31g
F = 154°C
Rendement = 68 %

B. 1-(CYCLOHEXYLAMINO CARBONYL N-PENTANOYL-4-(1H-IMIDAZOLYL-4) PIPERIDINE

Le composé obtenu sous A fournit le composé du titre en appliquant le même procédé que celui décrit dans l'exemple 2.B.

Exemple 7 :

1-(CYCLOPENTENE METHYL CARBONYL)-4-(1H-IMIDAZOLYL-4)PIPERIDINE (Composé 61)

On ajoute goutte à goutte à 0°C à une solution de 1,66g (0,0132 mole) d'acide cyclopenténe acétique dans 70 ml d'acétonitrile 2,03g (0,0132 mole) de triéthylamine. Après 30 minutes d'agitation, on ajoute 1,43g (0,0132 mole) de chloroformiate d'éthyle de telle façon que la température reste comprise entre 0°C et 5°C. Après 30 minutes d'agitation, la solution est versée dans 60 ml d'acétonitrile et 15 ml d'eau contenant 1,99g (0,0132 mole) de 4-(imidazolyl-4) pipéridine. Après 1 heure de chauffage à 80°C, la solution est concentrée sous pression réduite, le résidu huileux est repris dans 200 ml d'eau puis extrait par 70 ml d'acétate d'éthyle. L'huile résiduelle obtenue cristallise par addition d'un mélange éther éthylique/éther de pétrole (3/2). On obtient le composé du titre sous forme d'une poudre blanche (recristallisation dans éther/éther de pétrole (60/40)).
F = 107°C
Masse = 2G
Rendement = 58 %
Spectre IR (KBr) : 3130 (NH), 1640 (CO) $cm^{-1}$
Analyse : C, H, N.

Exemple 8 :

METHODE GENERALE DE SYNTHESE DES ISOCYANATES (Composés 67 à 75)

On ajoute à (x mole) d'acide en solution dans x ml d'acétonitrile à 0°C, (x mole) de triéthylamine ; après 30 mn de contact, on additionne goutte à goutte (x mole) de chloroformiate d'éthyle et on laisse sous agitation à cette température pendant 40 mn. (x mole) d'azoture de sodium dans (x ml) d'eau sont additionnés goutte à goutte en 10 mn à la solution précédente. Après 1H de contact, le précipité est essoré, et la solution filtrée est hydrolysée par 200 ml d'eau froide puis extraite par 200 ml d'éther éthylique. La phase éthérée est séchée sur sulfate de magnésium et chlorure de calcium. Après évaporation sous vide à 40°C, l'huile résiduelle est reprise par 60 ml de benzène et portée au reflux 2H. La solution benzénique est lavée à l'eau, décantée, séchée puis concentrée sous pression réduite. L'huile résiduelle est distillée sous 5 mm. Les isocyanates obtenus (Tableau II) se présentent sous forme d'huiles qui sont conservées sous azote et à l'abri de la lumière car ils forment très rapidement les urées symétriques.

Exemple 9 :

METHODE GENERALE DE SYNTHESE DES N-(4-IMIDAZOLYL)PIPERIDINYLUREES (OXALATES) (Composés 76 à 84 du TABLEAU III)

Une solution de 2,5g (0,016 mole) d'isocyanate et de 2,41g (0,016 mole) de 4-(imidazolyl-4)pipéridine dans 60 ml de benzène est chauffée au reflux 1H30. Après refroidissement de la solution, le benzène est décanté, l'huile résiduelle est reprise dans 60 ml d'acétone, agitée à 40°C, puis additionnée d'un léger excès d'acide oxalique. Le mélange est chauffé 35 mn à ébullition. Après refroidissement, le précipité formé est essoré, lavé à l'éther éthylique, séché et recristallisé. Les urées 76 à 84 décrites dans le TABLEAU III sont synthétisées de façon analogue.

Exemple 10 :

(PHENETHYL AMINO CARBOTHIOYL-1 PIPERIDINYL-4)-41H-IMIDAZOLE, (OXALATE) (Composé 85)

Une solution de 1,63g (0,01 mole) de phénéthyl isothiocyanate dans 60 ml de toluène et 1,51g (0,01 mole) de 4-(imidazolyl-4)pipéridine est chauffée 1H30 au reflux. Après refroidissement, la solution est évaporée sous pression réduite, l'huile résiduelle obtenue est mise en solution dans 60 ml d'isopropanol en présence de 1,2 équivalent d'acide oxalique. Après 30 minutes de chauffage à 80°C, le précipité blanc formé est essoré, lavé à l'éther et recristallisé dans l'acétonitrile.
F = 201°C
Masse = 2,7g
Rendement = 67 %

Analyse : $C_{19}H_{24}N_4O_4S$

Calculé % : C : 56,43 ; H : 5,90 ; N : 13,86

Trouvé % : C : 56,55 ; H : 5,91 ; N : 13,90

Exemple 11 :

1-(3-CYCLOPENTYLPROPIONYL)-4-[-1-(3-CYCLOPENTYLPROPIONYL-)-4- IMIDAZOLYL-)] PIPERIDINE (Composé 86)

1g (0,0036 mole) de 1-(3-cyclopentylpropionyl)-4-(1H-imidazolyl-4)pipéridine et 0,57g (0,0036 mole) de chlorure de l'acide 3-cyclopentylpropionique dans 70 ml de toluène sont portés aux reflux 1H en présence de 2 ml de triéthylamine. Après refroidissement, la solution est évaporée sous pression réduite, le résidu est repris par 100 ml d'eau puis extrait par 200 ml d'éther éthylique. On obtient le composé du titre sous forme de poudre blanche (recristallisation dans l'acétone).
Masse = 0,71g
Rendement = 50 %
Spectre IR (KBr) : (CO) 1620, 1650 $cm^{-1}$

Analyse : $C_{24}H_{37}N_3O_2$

Calculé % : C : 72,15 ; H : 9,30 ; N : 10,51

Trouvé % : C : 72,47 ; H : 9,23 ; N : 10,97

Les composés 87 et 88 (Tableau V) sont obtenus de façon analogue.

Exemple 12 :

1-(4-PHENOXYBUTYRYL)-4-[-1-(2,4-DICHLOROPHENYL AMINOCARBONYLIMIDAZOLYL-4)] PIPERIDINE (Composé 92)

1g (0,00319 mole) de 1-(4-phénoxybutyryl)-4-(1H-imidazolyl-4)pipéridine et 0,59g (0,00319 mole) de 2,4-dichlorophénylisocyanate dans 50 ml de toluène sont portés au reflux 2H. Le précipité formé est essoré et lavé à l'éther éthylique. On obtient le composé du titre sous forme de poudre blanche (recristallisation dans l'acétate d'éthyle).
F = 124°C
Masse = 0,51g
Rendement = 30 %
Spectre IR (KBr) : (CO) 1625 et 1640 $cm^{-1}$

Analyse : $C_{25}H_{26}N_4Cl_2$

Calculé % : C:58,94 ; H:5,14 ; N:10,99 ; Cl:13,91

Trouvé % : C:58,96 ; H:5,20 ; N:10,87 ; Cl:14,00

Les composés 89, 90, 91, 93 et 94 (Tableau V) sont obtenus de façon analogue.
Les composés des exemples précédents sont rassemblés dans les tableaux I à V suivants.

TABLEAU I

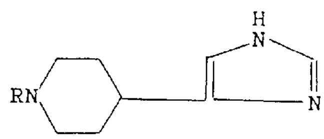 , HX

| Comp. N° | R | HX | F(°C) | RdT (%) | Analyse | Solvant de Recristallisation |
|---|---|---|---|---|---|---|
| 1 | $CH_3(CH_2)_5$ -CO- | $(CO_2H)_2$ | 86 | 29 | C,H,N | Acétate d'éthyle (3) Ether (2) |
| 2 | 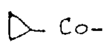 | | 160 | 76 | C,H,N | Acétate d'éthyle |
| 3 | 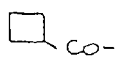 | | 153 | 53 | C,H,N | Acétate d'éthyle |
| 4 | 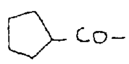 | | 165 | 86 | C,H,N | Acétate d'éthyle |
| 5 | 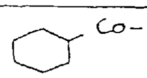 | | 106 | 71 | C,H,N | Ether éthylique |
| 6 | 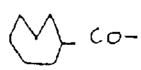 | $(CO_2H)_2$ | 100 | 60 | C,H,N | Ether éthylique |
| 7 | 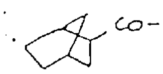 | | 158 | 50 | C,H,N | Acétate d'éthyle (3) Ether (2) |
| 8 | 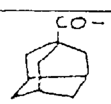 | $(CO_2H)_2$ | 108 | 57 | C,H,N | Isopropanol |
| 9 | 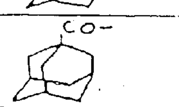 | $(CO_2H)_2$ | 167 | 55 | C,H,N | Isopropanol |
| 10 | 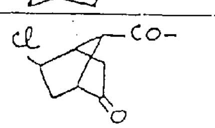 | $(CO_2H)_2$ | 168 | 45 | C,H,N | Acétate d'éthyle (3 Ether (2) |
| 11 | 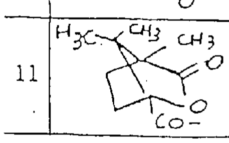 | | 164 | 40 | C,H,N | Acétate d'éthyle (3 Ether (2) |

TABLEAU I (suite)

| Comp. N° | R | HX | F (°C) | Rdt (%) | Analyse | Solvant de Recristallisation |
|---|---|---|---|---|---|---|
| 12 | 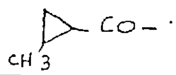 | | 126 | 60 | C,H,N | Acétone (4) Ether (1) |
| 13 | 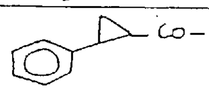 | | 144 | 60 | C,H,N | Acétate d'éthyle (3) Ether (2) |
| 14 | 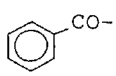 | | 150 | 65 | C,H,N | Acétone (4) Ether (1) |
| 15 | 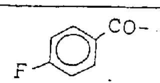 | $(CO_2H)_2$ | 158 | 40 | C,H,N,F | Acétone (3) Ether (2) |
| 16 | 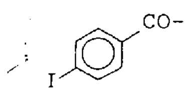 | | 198 | 55 | C,H,N,I | Acétate d'éthyle (3) Ether (2) |
| 17 | 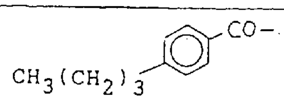 | | 151 | 70 | C,H,N | Acétonitrile (3) Ether (2) |
| 18 | 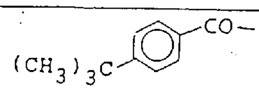 | | 174 | 80 | C,H,N | Acétate d'éthyle (3) Ether (1) |
| 19 | 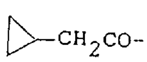 | $(CO_2H)_2$ | 115 | 20 | C,H,N | Acétate d'éthyle (3) Ether (2) |
| 20 | 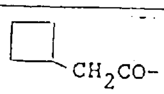 | | 128 | 60 | C,H,N | Ether éthylique |
| 21 | 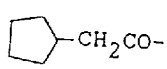 | | 122 | 41 | C,H,N | Ether éthylique |

TABLEAU I (suite)

| Comp. N° | R | HX | F (°C) | Rdt (%) | Analyse | Solvant de Recristallisation |
|---|---|---|---|---|---|---|
| 22 | 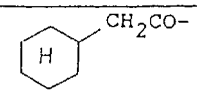 | | 164 | 70 | C,H,N | Acétonitrile |
| 23 | 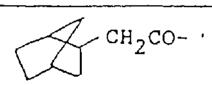 | | 118-120 | 60 | C,H,N | Ether éthylique |
| 24 | 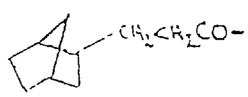 | | 158 | 65 | C,H,N | Acétate d'éthyle( Ether (2) |
| 25 | 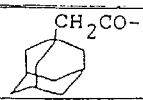 | | 118 | 55 | C,H,N | Acétone |
| 26 | 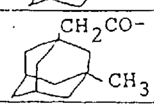 | | 134 | 30 | C,H,N | Ether éthylique |
| 27 | 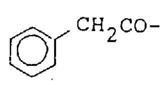 | | 188 | 45 | C,H,N | Ether(1) Acétate d'éthyle (2) |
| 28 | 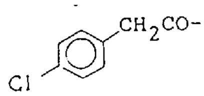 | | 157 | 20 | C,H,N | Ether (1),Acétate d' éthyle (1) Ether de pétrole (1). |
| 29 | 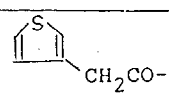 | | 142 | 30 | C,H,N,S | Ether éthylique |
| 30 | 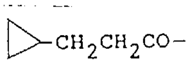 | | 109 | 10 | C,H,N | Acétate d'éthyle (3) Ether (2). |
| 31 | 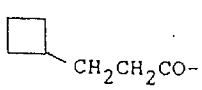 | | 124 | 10 | C,H,N | Acétate d'éthyle (2) Ether (2) |

TABLEAU I (suite)

| Comp. N° | R | HX | F (°C) | Rdt (%) | Analyse | Solvant de Recristallisation |
|---|---|---|---|---|---|---|
| 32 | 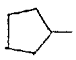 $CH_2CH_2CO-$ | | 116 | 73 | C,H,N | Ether éthylique |
| 33 | 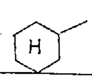 $CH_2CH_2CO-$ | | 110 | 68 | C,H,N | Ether éthylique |
| 34 | 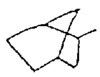 $CH_2CH_2CO-$ | | 158 | 65 | C,H,N | Acétate d'éthyle |
| 35 | $CH_2CH_2CO-$ 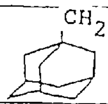 | | 134 | 30 | C,H,N | Acétone |
| 36 | $CH_2CH_2CO-$ 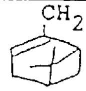 | | 100 | 15 | C,H,N | Ether éthylique |
| 37 | 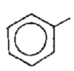 $CH_2CH_2CO-$ | | 121 | 40 | C,H,N | Ether (1),Acétate d'éthyle (2) |
| 38 | 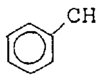 $CH_2CH_2CO-$ | $(CO_2H)_2$ | 129 | 94 | C,H,N | Acétonitrile |
| 39 | $CH_3O$ 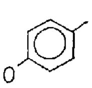 $CH_2CH_2CO-$ | | 123 | 63 | C,H,N | Ether (1)Ether de pétrole (1) |
| 40 | 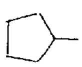 $-(CH_2)_3CO-$ | | 144 | 60 | C,H,N | Ether (1)Ether de pétrole (1) |
| 41 | 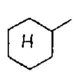 $(CH_2)_3CO-$ | | 146 | 60 | C,H,N | Acétate d'éthyle |
| 42 | 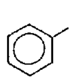 $(CH_2)_3CO-$ | | 102 | 81 | C,H,N | Ether (3) Hexane(2) |

TABLEAU I (suite)

| Comp. N° | R | HX | F (°C) | Rdt (%) | Analyse | Solvant de Recristallisation |
|---|---|---|---|---|---|---|
| 43 | 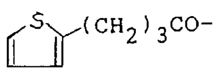 | | 103 | 70 | C,H,N,S | Ether éthylique |
| 44 | 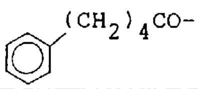 | | 103-105 | 65 | C,H,N | Ether éthylique |
| 45 | $CH_3(CH_2)_6CO-$ | | 100 | 45 | C,H,N | Ether éthylique (4)<br>Ether de pétrole(1) |
| 46 | $CH_3(CH_2)_8CO-$ | | 95 | 55 | C,H,N | Ether éthylique (4)<br>Ether de pétrole(1) |
| 47 | 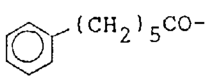 | | 102 | 56 | C,H,N | Ether (3)<br>Hexane (2) |
| 48 | 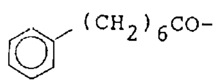 | | 92 | 65 | C,H,N | Ether (3),Hexane (1)<br>Ether de pétrole (1) |
| 49 | 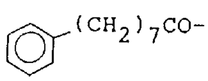 | | 120 | 75 | C,H,N | Ether éthylique |
| 50 | 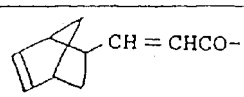 | $(CO_2H)_2$ | 136 | 45 | C,H,N | Isopropanol |
| 51 | 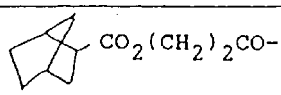 | | 189 | 70 | C,H,N | Ether (1),Acétate d'éthyle (4) |
| 52 | 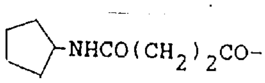 | | 175 | 20 | C,H,N | Acétone |

TABLEAU I (suite)

| Comp N° | R | HX | F°(C) | Rdt (%) | Analyse | Solvant de Recristallisation |
|---|---|---|---|---|---|---|
| 53 | 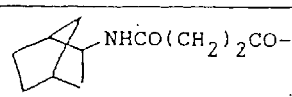 | | 124 | 40 | C,H,N | Acétate d'éthyle (3)<br>Ether (2) |
| 54 | 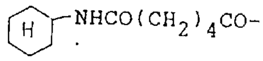 | | 121 | 35 | C,H,N | Ether éthylique |
| 55 | 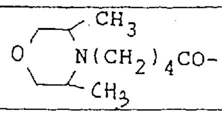 | | 122 | 20 | C,H,N | Acétone (4)<br>Ether (1) |
| 56 | 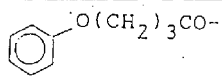 | | 115 | 43 | C,H,N | Ether éthylique |
| 57 | 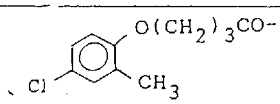 | | 155 | 60 | C,H,N | Acétonitrile |
| 58 | 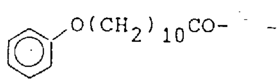 | | 103 | 45 | C,H,N | Ether éthylique |
| 59 | 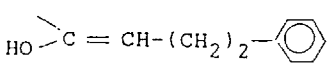 | HCl | 126 | 55 | C,H,N,Cl | Acétonitrile (7)<br>Ether éthylique (3) |
| 60 | 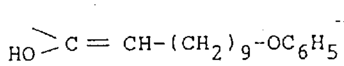 | HCl | 115 | 55 | C.H.N,Cl | Acétonitrile (7)<br>Ether éthylique (3) |
| 61 | 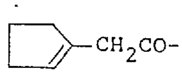 | | 107 | 58 | C,H,N | Ether (6)<br>Ether de pétrole (4) |

TABLEAU I (suite)

| Comp. N° | R | HX | F (°c) | Rdt % | Analyse | Solvant de Recristallisation |
|---|---|---|---|---|---|---|
| 62 | $-CO(CH_2)_4-CH_3$ | | 104 | 61 | C,H,N, | éther éthyliqu |
| 63 | $-CO(CH_2)_7-CH_3$ | | 96 | 52 | C,H,N, | éther éthyliqu Hexane 1/1 |
| 64 | $-CO(CH_2)_9-CH_3$ | | 114 | 48 | C,H,N, | éther éthyliqu |
| 65 | $-CO(CH_2)_{10}-CH_3$ | | 94 | 22 | C,H,N, | éther éthyliqu Hexane 1/1 |
| 66 | -CO- 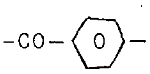 1 | $(CO_2H)_2$ | 118 | 73 | C,H,N, | éther éthyliqu Acetate d'éthy 1/4 |

TABLEAU II

$R_9(CH_2)_nNCO$

| Comp. N° | $R_9$ | n | Eb (5mm)(°C) | IR ( NCO) | Rdt % | Analyses |
|---|---|---|---|---|---|---|
| 67 | 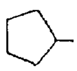 | 2 | 150 | 2190 | 20 | C,H,N |
| 68 | 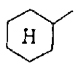 | 2 | 170 | 2180 | 25 | C,H,N |
| 69 | 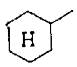 | 3 | 185 | 2180 | 30 | C,H,N |
| 70 | 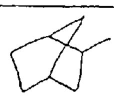 | 1 | 190 | 2190 | 40 | C,H,N |
| 71 | 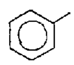 | 2 | 180 | 2185 | 20 | C,H,N |
| 72 | 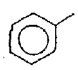 | 3 | 190 | 2180 | 20 | C,H,N |
| 73 | 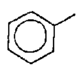 | 4 | 195 | 2180 | 25 | C,H,N |
| 74 | 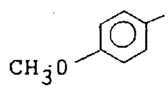 | 2 | 185 | 2190 | 20 | C,H,N |
| 75 | 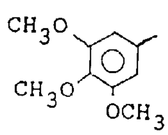 | 2 | 210 | 2190 | 20 | C,H,N |

TABLEAU III

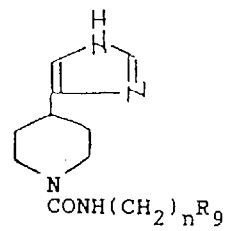

| Comp. N° | $R_9$ | n | F(°C) | Rdt % | Solvant de recristallisation |
|---|---|---|---|---|---|
| 76 | 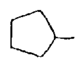 | 2 | 148 | 30 | Isopropanol |
| 77 | 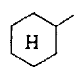 | 2 | 120 | 20 | Isopropanol |
| 78 | 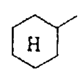 | 3 | 130 | 20 | Isopropanol |
| 79 | 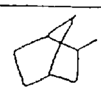 | 1 | 102 | 60 | Isopropanol |
| 80 | 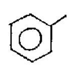 | 2 | 136 | 15 | Isopropanol: (6)<br>Ether éthylique : (4) |
| 81 | 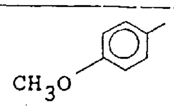 | 2 | 130 | 20 | Isopropanol: (3)<br>Ether éthylique: (3)<br>Hexane: (4) |
| 82 | 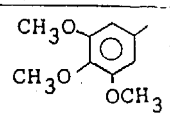 | 2 | 92 | 15 | Isopropanol: (6)<br>Ether éthylique: (4) |
| 83 | 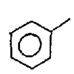 | 3 | 136 | 15 | Isopropanol (6)<br>Ether éthylique (4) |
| 84 | 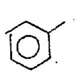 | 4 | 158 | 15 | Isopropanol |

TABLEAU IV

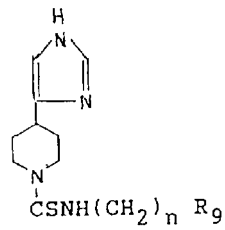

HX

| Comp. N° | $R_9$ | n | HX | F(°C) | RdT (%) | Solvant de Recristallisation |
|---|---|---|---|---|---|---|
| 85 | 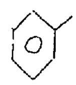 | 2 | $(CO_2H)_2$ | 201 | 67 | Acétonitrile |

TABLEAU V

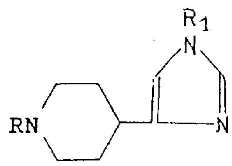

| Comp. N° | R | R1 | F (°C) | Rdt (%) | Analyse | Solvant de Recristallisation |
|---|---|---|---|---|---|---|
| 86 | 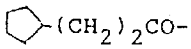 | 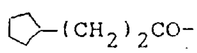 | 165 | 50 | C,H,N | Acétone |
| 87 | 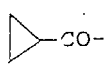 | 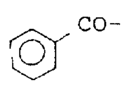 | 142 | 20 | C,H,N | Acétonitrile |
| 88 | 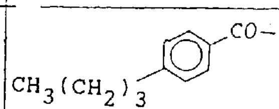 | 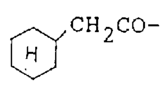 | 150 | 50 | C,H,N | Acétonitrile |
| 89 | 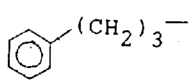 | 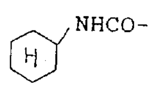 | 134 | 35 | C,H,N | Acétonitrile |
| 90 | 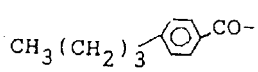 | 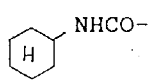 | 167 | 60 | C,H,N | Acétone (4)<br>Ether éthylique (1) |
| 91 | 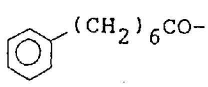 | 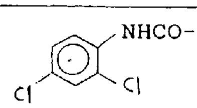 | 102 | 25 | C,H,N,Cl | Ether éthylique |
| 92 | 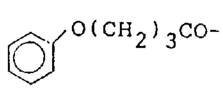 | 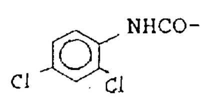 | 124 | 30 | C,H,N,Cl | Acétate d'éthyle |
| 93 | 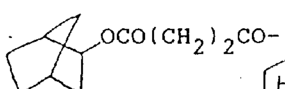 | 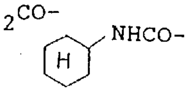 | 124 | 60 | C,H,N | Acétone (4)<br>Ether (1) |
| 94 | 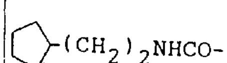 | 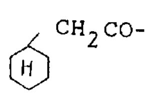 | 128 | 36 | C,H,N | Acétate d'éthyle |

ETUDE PHARMACOLOGIQUE

Les composés de formule I conformes à l'invention provoquent in vitro un blocage des récepteurs histaminergiques $H_3$ contrôlant la libération et la formation d'histamine cérébrale et in vivo une augmentation de la vitesse de renouvellement de l'histamine cérébrale, effets établissant notamment une action psychotrope.

L'antagonisme de la stimulation par l'histamine des récepteurs $H_3$ centraux a été mis en évidence grâce au procédé décrit par Arrang et coll. (Nature, 1983, 302 ; 832-837). Ce procédé fait appel à des coupes de cortex cérébral de rats et a permis la caractérisation pharmacologique des récepteurs $H_3$ (Nature, 1987, 327 ; 117-123).

L'histamine exogène (à la concentration de 1μM) produit une inhibition de libération d'environ 50%. Cet effet est progressivement reversé en présence d'antagonistes $H_3$ tels que les composés de l'invention, ajoutés en concentrations croissantes. La concentration de ces derniers pour laquelle l'effet de l'histamine exogène est réduit de moitié ($CI_{50}$) est déterminée et la constante apparente d'inhibition (Ki) est alors calculée suivant Cheng et Prusoff (Biochem. Pharmacol. 1973, 22, 3099-3108), en tenant compte de la concentration efficace 50% de l'histamine (CE = 0,1 μM). Les résultats sont rassemblés dans le tableau VI suivant.

TABLEAU VI : CONSTANTES APPARENTES DE DISSOCIATION (Ki) DE DIVERS DERIVES DE L'INVENTION COMME ANTAGONISTES DE L'HISTAMINE SUR LES RECEPTEURS $H_3$ DU CERVEAU DE RAT.

| COMPOSE N° | Ki (nM) |
|---|---|
| 1 | 48 |
| 8 | 63 |
| 13 | 20 |
| 20 | 59 |
| 23 | 23 |
| 38 | 84 |
| 43 | 35 |
| 48 | 65 |
| 59 | 47 |
| 60 | 120 |
| 77 | 68 |
| 83 | 77 |
| 92 | 34 |

Les composés de l'invention provoquent in vivo, après administration intrapéritonéale ou orale chez le rat à une dose inférieure ou égale à 30 mg/kg, une augmentation de la vitesse de renouvellement de l'histamine cérébrale. Cette dernière est estimée soit par l'étude de la décroissance du taux de l'histamine cérébrale après

blocage de sa synthèse (Garbarg et coll., Europ. Jr. Pharmacol. 164, 1-11, 1989), soit par l'étude de l'augmentation du taux du catabolite de l'histamine, la téléméthylhistamine (Garbarg et coll., J.Neurochem. 53, 1724-1730, 1989).

Cette propriété d'antagonistes -$H_3$ actifs par voie générale fait des composés de l'invention des dérivés utiles en médecine humaine et vétérinaire. Leurs applications thérapeutiques coccernent notamment le système nerveux central.

La présente invention concerne donc également les compositions pharmaceutiques qui contiennent à titre de principe actif, les composés de formule I.

La composition pharmaceutique conforme à l'invention est administrable à l'homme par voie orale, perlinguale, nasale, rectale et parentérale, le principe actif étant associé à un excipient ou véhicule thérapeutique convenable.

Chaque dose unitaire contient avantageusement de 0,5 mg à 100 mg de principe actif, les doses administrables journellement pouvant varier de 0,5 mg à 200 mg de principe actif.

## Revendications

**1.** Composés répondant à la formule générale

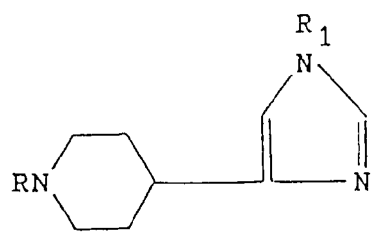

I

dans laquelle $R_1$ représente un atome d'hydrogène ou le groupement -$COR_2$ dans lequel $R_2$ représente le noyau benzénique, les groupements cyclopentylméthyle, cyclohexylméthyle, cyclopentyléthyle ou cyclohexyléthyle, les groupements cyclopentylamine, cyclohexylamine, phénylamine, chlorophénylamine ou dichlorophénylamine ; R représente un atome d'hydrogène ou le groupement $COR_3$ dans lequel $R_3$ représente

a) un groupement aliphatique linéaire ou ramifié comportant 1 à 11 et notamment 1 à 9 atomes de carbone,

b) un noyau cyclanique tel que cyclopropane, phénylcyclopropane, cyclobutane, cyclopentane, cyclohexane, cycloheptane, norbornane, adamantane, noradamantane, chlorooxonorbornane, chloroéthylène dioxynorbornane, bromoéthylène dioxynorbornane et le groupement anhydre de l'acide hydroxy carboxy 1,2,2-triméthylcyclopentane carboxylique,

c) un noyau benzénique non substitué ou substitué en para par un groupement aliphatique linéaire ou ramifié comportant 3 à 5 atomes de carbone ainsi que par un halogène,

d) un groupement $(CH_2)_nR_4$ dans lequel n est un nombre compris entre 1 et 10 et $R_4$ représente un noyau cyclanique tel que cyclopropane, cyclobutane, cyclopentane, cyclopentène, cyclohexane, cycloheptane, norbornane, noradamantane, adamantane et 6,6-diméthylbicyclo[3,1,1]heptène ; un cycle benzénique non substitué ou monoxubstitué par un atome de fluor, un atome de chlore, un groupement méthyle ou un groupement méthoxy; le cycle thiophénique greffé par son sommet 2 ou son sommet 3 ; un groupement ester d'acide carboxylique $COOR_5$ dans lequel $R_5$ est un noyau cyclanique tel que cyclopropane, cyclobutane, cyclopentane, cyclohexane ou norbornane ; un groupement amide d'acide carboxylique de structure $CONHR_6$ dans lequel $R_6$ représente un noyau cyclanique tel que cyclopropane, cyclobutane, cyclopentane, cyclohexane ou norbornane ; un groupement amide d'acide carboxylique de structure CON dans lequel le groupement N représente la pyrrolidine, la pipéridine, la 2,6-diméthylmorpholine ; un groupement éther oxyde -O-$R_7$, $R_7$ pouvant être un noyau benzénique non substitué ou monosubstitué par un atome de chlore ou de fluor ou disubstitué par un atome de chlore et par un groupement méthyle ;

e) un groupement -CH = $CHR_8$ dans lequel $R_8$ représente un noyau cyclanique tel que cyclopropane, cyclobutane, cyclopentane, cyclohexane, norbornane ou norbornène ;

f) le groupe amine secondaire -$NH(CH_2)_nR_9$ dans lequel n est un nombre compris entre 1 et 5 et $R_9$ constitue un noyau cyclanique tel que cyclopropane, noyau benzénique non substitué, monosubstitué

par un atome de fluor ou de chlore ou par un groupement méthoxy ou trisubstitué par les groupements méthoxy ; R représente encore le groupement hydroxy alkényle

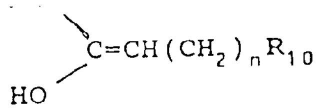

dans lequel n est un nombre compris entre 2 et 9 et $R_{10}$ représente le noyau benzénique ou le groupement phénoxy ; ainsi que le groupement

$$C\ S\ NH\ (CH_2)_nR_9$$

dans lequel n est un nombre compris entre 1 et 5 et $R_9$ a la signification précitée.

2. Composés selon la revendication 1, caractérosés en ce qu'ils sont choisis parmi :
   - la 1-(hexylcarbonyl)-4-(1H-imidazolyl-4) pipéridine,
   - la 1-(cyclopropylcarbonyl)-4-(1H-imidazolyl-4) pipéridine,
   - la 1-(cyclobutylcarbonyl)-4-(1H-imidazolyl-4) pipéridine,
   - la 1-(cyclopentylcarbonyl)-4-(1H-imidazolyl-4) pipéridine,
   - la 1-(cyclohexylcarbonyl)-4-(1H-imidazolyl-4) pipéridine,
   - la 1-(cycloheptylcarbonyl)-4-(1H-imidazolyl-4) pipéridine,
   - la 1-(norbornylcarbonyl)-4-(1H-imidazolyl-4) pipéridine,
   - la 1-(norbornylméthylcarbonyl)-4-(1H-imidazolyl-4) pipéridine,
   - la 1-(norbornyl propionyl carbonyl)-4-(4-1H-imidazolyl) pipéridine,
   - la 1-(adamantylcarbonyl)-4-(1H-imidazolyl-4) pipéridine,
   - la 1-(noradamantylcarbonyl)-4-(1H-imidazolyl-4) pipéridine,
   - la 1-(chlorooxonorbornylcarbonyl)-4-(1H-imidazolyl-4) pipéridine,
   - la 1-(méthylcyclopropylcarbonyl)-4-(1H-imidazolyl-4) pipéridine,
   - la 1-(phénylcyclopropylcarbonyl)-4-(1Himidazolyl-4) pipéridine,
   - la 1-(phénylcarbonyl)-4-(1H-imidazolyl-4) pipéridine,
   - la 1-(p-fluorophénylcarbonyl)-4-(1H-imidazolyl-4) pipéridine,
   - la 1-(p-iodophénylcarbonyl)-4-(1H-imidazolyl-4) pipéridine,
   - la 1-(p-butylphénylcarbonyl)-4-(1H-imidazolyl-4) pipéridine,
   - la 1-(p-t-butylphénylcarbonyl)-4-(1H-imidazolyl-4) pipéridine,
   - la 1-(3-phénylpropionyl)-4-(4-1H-imidazolyl) pipéridine,
   - la 1-(cyclohexylaminocarbonyl n-pentanoyl)-4-(1H-imidazolyl-4)pipéridine,
   - la 1-(thiophénylpropylcarbonyl)-4-(1H-imidazolyl-4) pipéridine,
   - la 1[1′-(1′-hydroxy-4′-phénylbuten-1′-yl)]-4-(4′-imidazolyl) pipéridine,
   - la 1[1′-(1′-hydroxy-11′-phénoxyundecen-1′-yl)]-4-(4′-imidazolyl) pipéridine,
   - la 1-(cyclopentène méthylcarbonyl)-4-(1H-imidazolyl-4)pipéridine,
   - la 1-cyclohexyléthylaminocarbonyl)-4-(1H-imidazolyl-4)pipéridine,
   - la 1-(phénylpropylaminocarbonyl)-4-(1H-imidazolyl-4)pipéridine,
   - la 1-(phénéthyl amino carbothioyl-1 pipéridinyl-4)-4 1H imidazole,
   - la 1-(3-cyclopentylpropionyl)-4-[-1-(3-cyclopentylpropionyl)-4-imidazolyl]pipéridine
   - la 1-(4-phénoxybutyryl)-4-[1-(2,4-dichlorophényl aminocarbonylimidazolyl-4) pipéridine.

3. Procédé de préparation des composés de formule générale I dans laquelle $R_1$ désigne l'hydrogène et R a la signification donnée dans la revendication 1, caractérisé en ce que l'on fait réagir la 4-(4-imidazolyl)pipéridine de formule

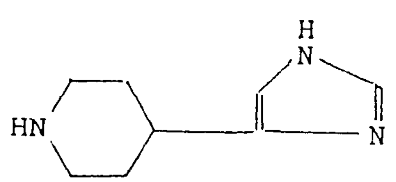

II

a) avec un chlorure d'acide $R_3$ COCl ou un anhydride mixte tel que $R_3CO_2CO_2C_2H_5$, lorsqu'il s'agit

d'obtenir des amides de formule

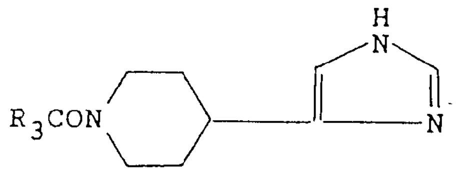

III

ou

b) lorsqu'il s'agit de préparer des urées de formule IV ou des thiourées de formule V

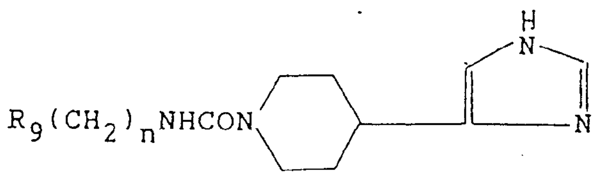

IV

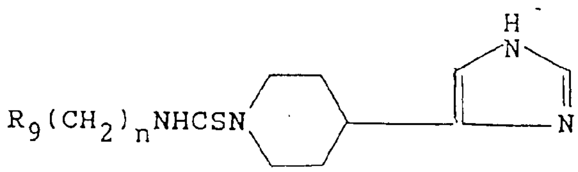

V

où n et $R_9$ ont la signification donnée dans la revendication 1, avec un isocyanate $R_9$ $(CH_2)_nNCO$ obtenu à partir d'un acide $R_9$ $(CH_2)_nCOOH$ par une modification de la synthèse de Curtius, ou avec un isothiocyanate $R_9$ $(CH_2)_nNCS$.

**4.** Procéde de préparation des composés de formule génerale I dans laquelle $R_1$ désigne le groupement $COR_2$ et R a la signification donnée dans la revendication 1, caractérisé en ce que l'on fait réagir un composé de formule

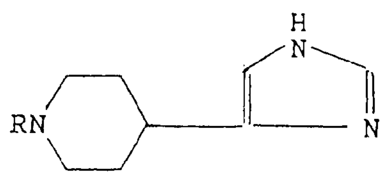

VI

a) avec un chlorure d'acide $R_2COCl$ lorsqu'il s'agit d'obtenir un composé de formule

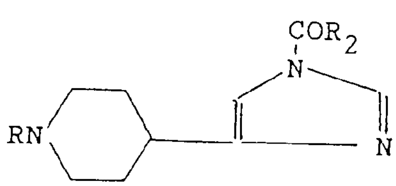

VII

où $R_2$ représente le noyau benzènique ou des groupements cyclopentylméthyle, cyclohexylméthyle, cyclopentyléthyle ou cyclohexyléthyle ;

ou

b) avec l'isocyanate correspondant lorsqu'il s'agit d'obtenir un composé de formule VII où $R_2$ représente les groupements cyclopentylamine, cyclohexylamine, phénylamine, chlorophénylamine ou dichlorophénylamine.

5. Composition pharmaceutique contenant un composé selon l'une des revendications 1 et 2 et un excipient ou véhicule thérapeutiquement compatible.

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 91 40 3498

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 197 840 (INSTITUT NATIONAL DE LA SANTE ET DE LA RECHERCHE MEDICALE)<br>* Pages 1-2 * | 1,5 | C 07 D 401/04<br>C 07 D 409/14<br>A 61 K 31/38<br>A 61 K 31/415<br>A 61 K 31/445 |
| X | CHEMICAL ABSTRACTS, vol. 80, no. 15, 15 avril 1974, page 389, abrégé no. 82801a, Columbus, Ohio, US; W. SCHUNACK: "Structure-action relationship of histamine analogs. 1. Histamine-like compounds with cyclized side chain", & ARCH. PHARM. (WEINHEIM, GER.) 1973, 306(12), 934-42<br>* Abrégé * | 1 | |
| A | US-A-4 431 653 (F. HOFFMANN - LA ROCHE & CO.) | | |
| | | | **DOMAINES TECHNIQUES RECHERCHES (Int. Cl.5)** |
| | | | C 07 D<br>A 61 K |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes: 2
Revendications ayant fait l'objet de recherches incomplètes: 1,3-5
Revendications n'ayant pas fait l'objet de recherches:
Raison pour la limitation de la recherche:

La rédaction des revendications n'est pas claire ni concise (Art. 83-84 OEB), et représente une telle masse énorme de produits, qu'une recherche complète n'est pas possible pour des raisons d'économie (voir Directives relatives à l'examen pratique à l'OEB, Partie B, (Chapitre III, 2). De cette facon la recherche a été fondé (règle 45) sur les composés, qui sont bien caractérisés par ses éléments physiques ou chimiques, c.a.d. les composés des exemples.

| Lieu de la recherche | Date d'achèvement de la recherche | Examinateur |
|---|---|---|
| LA HAYE | 27-03-1992 | DE BUYSER I.A.F. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0408)